(19) Europäisches Patentamt
European Patent Office
Office européen des brevets

(11) Veröffentlichungsnummer: **0 104 342**
**B1**

# (12) EUROPÄISCHE PATENTSCHRIFT

(45) Veröffentlichungstag der Patentschrift: **28.01.87**

(51) Int. Cl.⁴: **C 07 D 233/70**, A 61 K 31/415

(21) Anmeldenummer: **83107048.7**

(22) Anmeldetag: **19.07.83**

(54) **Triphenylimidazolyloxyalkansäuren und ihre Derivate, Verfahren zu ihrer Herstellung und diese enthaltende pharmazeutische Präparate.**

(30) Priorität: **29.07.82 DE 3228271**

(43) Veröffentlichungstag der Anmeldung: **04.04.84 Patentblatt 84/14**

(45) Bekanntmachung des Hinweises auf die Patenterteilung: **28.01.87 Patentblatt 87/5**

(84) Benannte Vertragsstaaten: **AT BE CH DE FR GB IT LI LU NL SE**

(56) Entgegenhaltungen:
**EP - A - 0 051 829**
**DE - A - 2 950 478**
**GB - A - 2 057 427**

**Die Akte enthält technische Angaben, die nach dem Eingang der Anmeldung eingereicht wurden und die nicht in dieser Patentschrift enthalten sind.**

(73) Patentinhaber: **A. Nattermann & Cie. GmbH, Nattermannallee 1, D-5000 Köln 30 (DE)**

(72) Erfinder: **Lautenschläger, Hans Heiner, Dr., Neusser Gasse 50, D-5024 Pulheim-Stommeln (DE)**
Erfinder: **Welter, André, Dr., Reiherweg 11a, D-5024 Pulheim (DE)**
Erfinder: **Hilboll, Gerd, Dr., Dehmelstrasse 36, D-5000 Köln 30 (DE)**
Erfinder: **Winkelmann, Johannes, Dr., Frankfurter Strasse 269, D-5000 Köln 90 (DE)**
Erfinder: **Prop, Gerrit, Dr., Mohnblumenweg 25, D-5024 Pulheim (DE)**
Erfinder: **Brekle, Axel, Dr., Gutenbergstrasse 33, D-5010 Bergheim (DE)**
Erfinder: **Zierenberg, Ottfried, Dr., Zum Dammfelde 45, D-5000 Köln 40 (DE)**

(74) Vertreter: **Redies, Bernd, Dr. rer. nat., COHAUSZ & FLORACK Patentanwaltsbüro Schumannstrasse 97 Postfach 14 01 47, D-4000 Düsseldorf 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft neue Triphenylimidazolyloxyalkansäuren und ihre Derivate, Verfahren zu ihrer Herstellung sowie ihre Verwendung als Wirkstoff in Arzneimitteln.

Einige N-substituierte omega-(2-oxo-4-imidazolin-1-yl)alkansäure Derivaten, die antithrombotische, entzündungshemmende und antiatherosklerotische Eigenschaften besitzen, sind aus EP-A 51 829 schon bekannt.

Die erfindungsgemässen Verbindungen entsprechend der allgemeinen Formel I

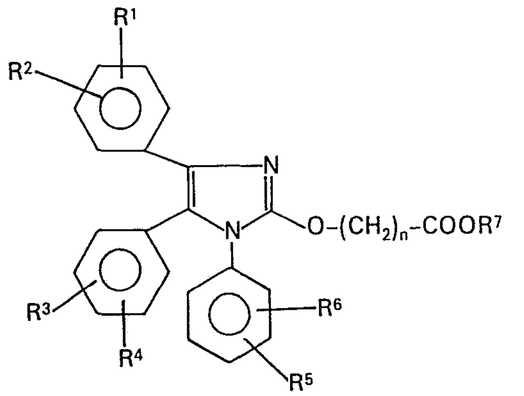

I

worin n eine ganze Zahl von 1–10 bedeutet, während $R^1$, $R^2$, $R^3$, $R^4$, $R^5$, $R^6$ gleich oder verschieden sein können und unabhängig voneinander Wasserstoff, Halogen, Alkyl, Alkoxy, Trifluormethyl oder 2 Reste zusammen Methylendioxy, darstellen, wobei neben Wasserstoff als Reste Methyl, Ethyl, n- oder Iso-propyl, Fluor, Chlor, Brom, Methoxy, Ethoxy besonders in Betracht kommen. $R^7$ bedeutet Wasserstoff, ein Alkaliion oder eine gradkettige bzw. verzweigte Alkylgruppe mit 1–6 Kohlenstoffatomen wie z.B. Methyl, Ethyl, Propyl, Isopropyl, Butyl, Isobutyl, oder einen Benzylrest, wobei die Reste Methyl bzw. Ethyl als Alkylreste bevorzugt sind.

Erfindungsgemässe Verbindungen sind beispielsweise:

1,4,5-Triphenylimidazol-2-yloxyessigsäure,
4-(1,4,5-Triphenylimidazol-2-yloxy)-buttersäure,
5-(1,4,5-Triphenylimidazol-2-yloxy)-valeriansäure,
6-(1,4,5-Triphenylimidazol-2-yloxy)-capronsäure,
7-(1,4,5-Triphenylimidazol-2-yloxy)-önanthsäure,
8-(1,4,5-Triphenylimidazol-2-yloxy)-caprylsäure,
8-(1,4,5-Triphenylimidazol-2-yloxy)-caprylsäuremethylester,
8-(1,4,5-Triphenylimidazol-2-yloxy)-caprylsäurethylester,
8-[4,5-Diphenyl-1-(4-methoxyphenyl)-imidazol-2-yloxy)-caprylsäure,
8-[1-(4-Chlorphenyl)-4,5-diphenylimidazol-2-yloxy]-caprylsäure,
8-[4,5-Diphenyl-1-(4-methylphenyl)-imidazol-2-yloxy]-caprylsäure,
8-[4,5-Diphenyl-1-(2-fluorphenyl)-imidazol-2-yloxy]-caprylsäure,
8-[4,5-Bis-(4-chlorphenyl)-1-phenyl-imidazol-2-yloxy]-caprylsäure,
8-[4,5-Bis-(4-fluorphenyl)-1-phenyl-imidazol-2-yloxy]-caprylsäure,
8-[4,5-Bis-(4-methoxyphenyl)-1-phenyl-imidazol-2-yloxy]-caprylsäure,
8-[1,4,5-Tris-(4-chlorphenyl)-imidazol-2-yloxy]-caprylsäure,
8-[1-(3,4-Dimethoxyphenyl)-4,5-diphenyl-imidazol-2-yloxy]-caprylsäure,
9-(1,4,5-Triphenylimidazol-2-yloxy)-pelargonsäure,
10-(1,4,5-Triphenylimidazol-2-yloxy)-caprinsäure,
11-(1,4,5-Triphenylimidazol-2-yloxy)-undecansäure.

Die erfindungsgemässen Verbindungen zeigen interessante pharmakologische Eigenschaften, insbesondere antithrombotische, entzündungshemmende, antiatherosklerotische und lipidsenkende Wirksamkeit bei ausgezeichneter Verträglichkeit. Sie können dementsprechend insbesondere zur Behandlung von thromboembolischen, entzündlichen, atherosklerotischen und mit dem Lipidstoffwechsel zusammenhängenden Krankheiten eingesetzt werden. Daneben besitzen die Verbindungen der Formel I den Vorteil einer geringen Toxizität.

Gegenstand der Erfindung sind weiterhin Verfahren zur Herstellung sowie pharmazeutische Zubereitungen dieser Verbindungen und ihre Verwendung als Wirkstoff für Arzneimittel.

Die erfindungsgemässen Verbindungen werden dadurch hergestellt, dass man ein 4-Imidazolin-2-on der allgemeinen Formel II

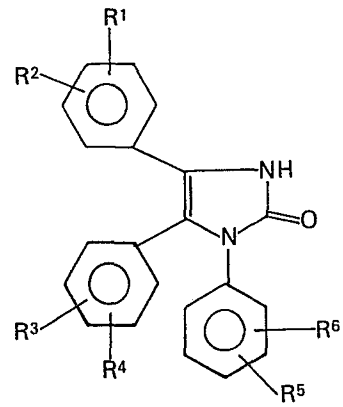

II

worin $R^1$, $R^2$, $R^3$, $R^4$, $R^5$, $R^6$ die in Formel I angegebenen Bedeutungen besitzen, in einem indifferenten organischen Lösungsmittel, wie z.B. Dimethylformamid, N,N-Dimethylacetamid durch Zusatz einer Hilfsbase wie z.B. Natriumhydrid, Kaliumhydrid oder Lithium organischer Verbindungen in das entsprechende Alkalisalz überführt und dieses mit einem Alkylierungsmittel der allgemeinen Formel III umsetzt,

$X-(CH_2)_n-COOR^7$ III

worin n und $R^7$ die in Formel I angegebenen Bedeutungen besitzen und X ein Halogen oder ein

Tosylrest ist. Die erhaltenen Ester werden z.B. durch Säulenchromatographie oder Umkristallisation von den infolge N-Alkylierung von II entstehenden Isomeren getrennt.

Die Ausgangsverbindungen der Formel II werden nach oder analog den bekannten Verfahren hergestellt, z.B. Org. Synth. Coll. Vol. II, 231

H. Ahlbrecht und H. Hanisch, Synthesis 1973, 109

H.G. Aurich, Liebigs Ann. Chem. 732, 195 (1970)

B. Krieg u. H. Lautenschläger, Liebigs Ann. Chem. 1976, 208

B. Krieg und H. Lautenschläger, Liebigs Ann. Chem. 1976, 1471

Y.A. Baskakov et al., USSR-Pat. 389 096 C.A. 79, 126 502 (1973)

Die erhaltenen Ester der Formel I können nach den üblichen Verfahren, z.B. durch Reaktion mit einem Alkalihydroxid in wässrigen, wässrig-organischen oder organischen Reaktionsmedien, wie z.B. Wasser, Alkoholen oder Ethern oder deren Mischungen, in das entsprechende Alkalisalz der Formel I und durch nachfolgenden Zusatz einer Mineralsäure in die Säuren der Formel I überführt werden.

Umgekehrt lassen sich aus den Säuren der Formel I und den Alkalisalzen der Formel I nach den in der organischen Chemie üblichen Verfahren die Ester der Formel I herstellen, so z.B. durch Behandeln der Säuren mit den entsprechenden Alkoholen unter Zusatz eines Kondensationsmittels wie Dicyclohexylcarbodiimid, z.B. durch Umesterung mit Ameisensäure- oder Essigsäureestern oder durch Alkylieren der Alkalisalze der Formel I mit den entsprechenden Alkylhalogeniden, Alkylsulfaten usw. in indifferenten Lösungsmitteln.

Die Herstellung der Verbindungen der Formel I wird durch das folgende Formelschema veranschaulicht:

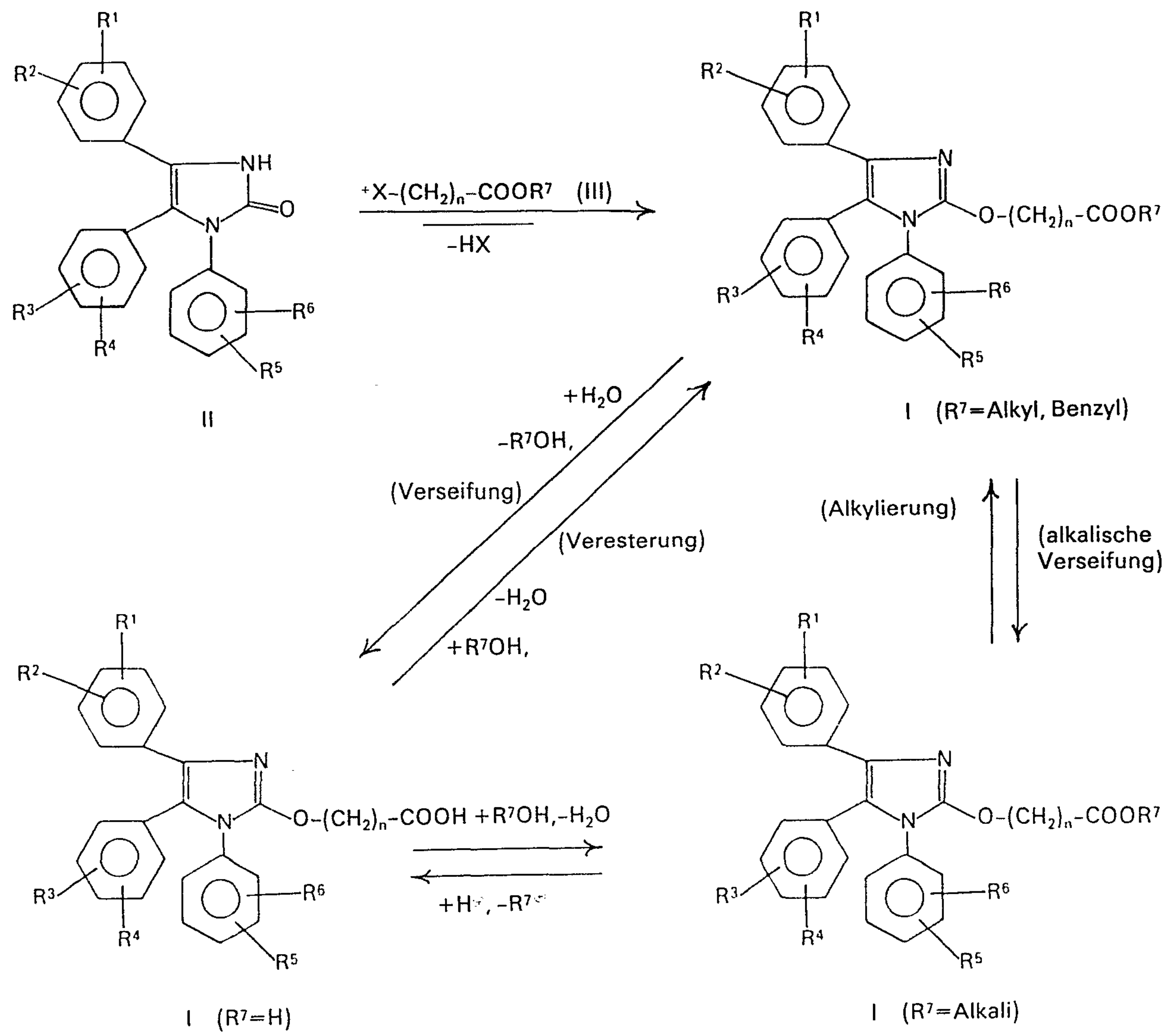


II

I ($R^7$=Alkyl, Benzyl)

I ($R^7$=H)

I ($R^7$=Alkali)

Als mit den Resten $R^1$, $R^2$, $R^3$, $R^4$, $R^5$ und $R^6$ substituierte Phenylradikale kommen in den Verbindungen I z.B. in Frage: Phenyl, 2-Fluorphenyl, 3-Fluorphenyl, 4-Fluorphenyl, 2-Chlorphenyl, 3-Chlorphenyl, 4-Chlorphenyl, 2-Methoxyphenyl, 3-Methoxyphenyl, 4-Methoxyphenyl, 2-Ethoxyphenyl, 3-Ethoxyphenyl, 4-Ethoxyphenyl, 2-Methylphenyl, 3-Methylphenyl, 4-Methylphenyl,

2-Trifluormethylphenyl, 3-Trifluormethylphenyl, 4-Trifluormethylphenyl, 2,3-Difluorphenyl, 2,4-Difluorphenyl, 2,5-Difluorphenyl, 2,6-Difluorphenyl, 3,4-Difluorphenyl, 3,5-Difluorphenyl, 2,3-Dichlorphenyl, 2,4-Dichlorphenyl, 2,5-Dichlorphenyl, 2,6-Dichlorphenyl, 3,4-Dichlorphenyl, 3,5-Dichlorphenyl, 2,3-Dimethoxyphenyl, 2,4-Dimethoxyphenyl, 2,5-Dimethoxyphenyl, 2,6-Dimethoxyphenyl, 3,4-Dimethoxyphenyl, 3,5-Dimethoxyphenyl, 2,3-Dimethylphenyl, 2,4-Dimethylphenyl, 2,5-Dimethylphenyl, 2,6-Dimethylphenyl, 3,4-Dimethylphenyl, 3,5-Dimethylphenyl, 3,4-Methylendioxyphenyl.

Als Alkylierungsmittel der Formel III kommen z.B. die Ester folgender ω-Halogenalkansäuren in Frage:

Chloressigsäure, Bromessigsäure, Jodessigsäure, 3-Chlorpropionsäure, 3-Brompropionsäure, 3-Jodpropionsäure, 4-Chlorbuttersäure, 4-Brombuttersäure, 4-Jodbuttersäure, 5-Chlorvaleriansäure, 5-Bromvaleriansäure, 5-Jodvaleriansäure, 6-Chlorcapronsäure, 6-Bromcapronsäure, 6-Jodcapronsäure, 7-Chlorönanthsäure, 7-Bromönanthsäure, 7-Jodönanthsäure, 8-Chlorcaprylsäure, 8-Bromcaprylsäure, 8-Jodcaprylsäure, 9-Chlorpelargonsäure, 9-Brompelargonsäure, 9-Jodpelargonsäure, 10-Chlorcaprinsäure, 10-Bromcaprinsäure, 10-Jodcaprinsäure, 11-Chlorundecansäure, 11-Bromundecansäure, 11-Jodundecansäure. Bei den Alkoholen $R^7OH$ handelt es sich vorzugsweise um solche mit geradkettigem oder sekundär- verzweigtkettigem gesättigtem Kohlenwasserstoffrest mit 1-6 Kohlenstoffatomen wie z.B. Methanol, Ethanol, Propanol, Isopropanol, Butanol, Pentanol, Hexanol sowie um Benzylalkohol.

Als Beispiele für Alkylierungsmittel zur Überführung der Alkalisalze I in die entsprechenden Ester seien genannt: Diazomethan, Dimethylsulfat, Chlormethan, Brommethan, Jodmethan, Chlorethan, Bromethan, Jodethan, Benzylchlorid, Benzylbromid.

Die vorliegende Erfindung betrifft ebenfalls pharmazeutische Präparate, welche die neuen Triphenylimidazolyloxyalkansäurederivate in Form ihrer freien Säuren oder als Salze mit pharmakologisch verträglichen Basen oder in Form ihrer Ester enthalten. Bei den erfindungsgemässen pharmazeutischen Präparaten handelt es sich um solche zur enteralen wie oralen oder rektalen sowie parenteralen Verabreichung, welche die pharmazeutischen Wirkstoffe allein oder zusammen mit einem üblichen, pharmazeutisch anwendbaren Trägermaterial enthalten. Vorteilhafterweise liegt die pharmazeutische Zubereitung des Wirkstoffes in Form von Einzeldosen vor, die auf die gewünschte Verabreichung abgestimmt sind, wie z.B. Tabletten, Dragees, Kapseln, Suppositorien, Granulate, Lösungen, Emulsionen oder Suspensionen. Die Dosierung der Verbindungen liegt üblicherweise zwischen 1–1000 mg pro Dosis, vorzugsweise zwischen 10–100 mg je Dosis und kann ein- oder mehrmals, bevorzugt zwei- bis dreimal täglich, verabreicht werden.

Die Herstellung der erfindungsgemässen Verbindungen wird durch die folgenden Beispiele näher erläutert.

Die angegebenen Schmelzpunkte wurden mit einem Büchi 510-Schmelzpunktbestimmungsapparat gemessen und sind nicht korrigiert. Die IR-Spektren wurden mit dem Gerät Nicolet NIC-3600 und die Massenspektren mit dem Gerät Varian MAT-311A (70 eV) aufgenommen.

Beispiel 1

Darstellung von 8-(1,4,5-Triphenylimidazol-2-yloxy)-caprylsäuremethylester.

18 g 80%ige Natriumhydrid-Mineralölsuspension werden mit n-Pentan gewaschen und zu einer Mischung aus 189 g 1,4,5-Triphenyl-4-imidazolin-2-on in 1200 ml trockenem Dimethylformamid hinzugefügt. Die Mischung wird erst bei Raumtemperatur, dann unter Rückfluss bis zum Ende der Wasserstoffentwicklung gerührt. Bei Rückflusstemperatur werden 142 g 8-Bromoctansäure-methylester zugetropft. Die Mischung wird ca. 3 Stunden bei dieser Temperatur weitererhitzt, nach dem Abkühlen mit Wasser verdünnt und mit Chloroform extrahiert. Die Chloroformlösung wird nacheinander mit Wasser, 5%iger Natriumhydrogencarbonatlösung und Wasser gewaschen, über Natriumsulfat getrocknet und im Vakuum eingeengt. Der Rückstand wird durch Säulenchromatographie (Kieselgel//Hexan/Essigsäureethylester) gereinigt.

Ausbeute: 58 g mit Fp. 85°C IR (in KBr): 1740 $cm^{-1}$

Beispiel 2

Darstellung von 4-(1,4,5-Triphenylimidazol-2-yloxy)-buttersäureethylester analog Beispiel 1 aus:

36,5 g 35%ige Kaliumhydrid-Mineralölsuspension,
100 g 1,4,5-Triphenyl-4-imidazolin-2-on,
850 ml Dimethylformamid,
48 g 4-Chlorbuttersäureethylester.

Reinigung durch Säulenchromatographie (Kieselgel//Hexan/Essigsäureethylester)

Ausbeute: 23,8 g mit Fp. 107–109°C IR (in KBr): 1740 $cm^{-1}$

Beispiel 3

Darstellung von 5-(1,4,5-Triphenylimidazol-2-yloxy)-valeriansäureethylester analog Beispiel 1 aus:

9,6 g 80%ige Natriumhydrid-Mineralölsuspension,
100 g 1,4,5-Triphenyl-4-imidazolin-2-on,
500 ml Dimethylformamid,
67 g 5-Bromvaleriansäurethylester.

Reinigung durch Säulenchromatographie (Kieselgel//Hexan/Essigsäureethylester)

Ausbeute: 19,6 g mit Fp. 84°C IR (in KBr): 1724 $cm^{-1}$

Beispiel 4

Darstellung von 6-(1,4,5-Triphenylimidazol-2-yloxy)-capronsäureethylester analog Beispiel 1 aus:

33,9 g 35%ige Kaliumhydrid-Mineralölsuspension,
93 g 1,4,5-Triphenyl-4-imidazolin-2-on,
850 ml Dimethylformamid,
66,9 g 6-Bromcapronsäureethylester.
Reinigung durch Säulenchromatographie (Kieselgel//Hexan/Essigsäureethylester)
Ausbeute: 28,6 g mit Fp. 102–103°C IR (in KBr): 1726 $cm^{-1}$

Beispiel 5
Darstellung von 7-(1,4,5-Triphenylimidazol-2-yloxy)-önanthsäureethylester analog Beispiel 1 aus:
3,7 g 35%ige Kaliumhydrid-Mineralölsuspension,
10 g 1,4,5-Triphenyl-4-imidazolin-2-on,
300 ml Dimethylformamid,
7,6 g 7-Chlorönanthsäureethylester.
Reinigung durch Säulenchromatographie (Kieselgel//Hexan/Essigsäureethylester)
Ausbeute: 4,3 g mit Fp. 78–81°C IR (in KBr): 1727 $cm^{-1}$

Beispiel 6
Darstellung von 8-[4,5-Diphenyl-1-(4-methoxyphenyl)-imidazol-2-yloxy]-caprylsäuremethylester analog Beispiel 1 aus:
17,6 g 35%ige Kaliumhydrid-Mineralölsuspension,
53 g 4,5-Diphenyl-1-(4-methoxyphenyl)-4-imidazolin-2-on,
300 ml Dimethylformamid,
37 g 8-Bromcaprylsäuremethylester.
Reinigung durch Säulenchromatographie (Kieselgel//Hexan/Essigsäureethylester)
Ausbeute: 25 g mit Fp. 68–70°C IR (in KBr): 1732 $cm^{-1}$

Beispiel 7
Darstellung von 8-[1-(4-Chlorphenyl)-4,5-diphenylimidazol-2-yloxy]-caprylsäuremethylester analog Beispiel 1 aus:
18,8 g 35%ige Kaliumhydrid-Mineralölsuspension,
57 g 1-(4-Chlorphenyl)-4,5-diphenyl-4-imidazolin-2-on,
300 ml Dimethylformamid,
39 g 8-Bromcaprylsäuremethylester.
Reinigung durch Säulenchromatographie (Kieselgel//Hexan/Essigsäureethylester)
Ausbeute: 17,2 g mit Fp. 89–91°C IR (in KBr): 1734 $cm^{-1}$

Beispiel 8
Darstellung von 8-[4,5-Diphenyl-1-(4-methylphenyl)-imidazol-2-yloxy]-caprylsäuremethylester analog Beispiel 1 aus:
26 g 35%ige Kaliumhydrid-Mineralölsuspension,
54 g 4,5-Diphenyl-1-(4-methylphenyl)-4-imidazolin-2-on,
400 ml Dimethylformamid,
72 g 8-Bromcaprylsäuremethylester.
Reinigung durch Säulenchromatographie (Kieselgel//Hexan/Essigsäureethylester)
Ausbeute: 11 g mit Fp. 108–110°C IR (in KBr): 1746 $cm^{-1}$

Beispiel 9
Darstellung von 8-[4,5-Diphenyl-1-(2-fluorphenyl)-imidazol-2-yloxy]-caprylsäuremethylester analog Beispiel 1 aus:
6,8 g 80%ige Natriumhydrid-Mineralölsuspension,
74 g 4,5-Diphenyl-1-(2-fluorphenyl)-4-imidazolin-2-on,
500 ml Dimethylformamid,
53 g 8-Bromcaprylsäuremethylester.
Reinigung durch Säulenchromatographie (Kieselgel//Hexan/Essigsäureethylester)
Ausbeute: 21 g mit Fp. 78°C IR (in KBr): 1736 $cm^{-1}$

Beispiel 10
Darstellung von 8-[4,5-Bis-(4-chlorphenyl)-1-phenyl-imidazol-2-yloxy]-caprylsäuremethylester analog Beispiel 1 aus:
5,6 g 80%ige Natriumhydrid-Mineralölsuspension,
70 g 4,5-Bis-(4-chlorphenyl)-1-phenyl-4-imidazolin-2-on,
500 ml Dimethylformamid,
44 g 8-Bromcaprylsäuremethylester.
Reinigung durch Säulenchromatographie (Kieselgel//Hexan/Essigsäureethylester)
Ausbeute: 14,7 g mit Fp. 92–95°C IR (in KBr): 1738 $cm^{-1}$

Beispiel 11
Darstellung von 8-[4,5-Bis-4-fluorphenyl)-phenyl-imidazol-2-yloxy]-caprylsäuremethylester analog Beispiel 1 aus:
19,8 g 35%ige Kaliumhydrid-Mineralölsuspension,
60 g 4,5-Bis-(4-fluorphenyl)-1-phenyl-4-imidazolin-2-on,
700 ml Dimethylformamid,
41 g 8-Bromcaprylsäuremethylester.
Reinigung durch Säulenchromatographie (Kieselgel//Hexan/Essigsäureethylester)
Ausbeute: 18,6 g mit Fp. 126–128°C IR (in KBr): 1740 $cm^{-1}$

Beispiel 12
Darstellung von 8-[4,5-Bis-(4-methoxyphenyl)-1-phenylimidazol-2-yloxy]-caprylsäuremethylester analog Beispiel 1 aus:
41,5 g 35%ige Kaliumhydrid-Mineralölsuspension,
135 g 4,5-Bis-(4-methoxyphenyl)-1-phenyl-4-imidazolin-2-on,
800 ml Dimethylformamid,
86 g 8-Bromcaprylsäuremethylester.
Reinigung durch Säulenchromatographie (Kieselgel//Hexan/Essigsäureethylester)
Ausbeute: 19 g mit Fp. 70–72°C IR (in KBr): 1738 $cm^{-1}$

Beispiel 13
Darstellung von 8-[1,4,5-Tris-(4-chlorphenyl)-imidazol-2-yloxy]-caprylsäuremethylester analog

Beispiel 1 aus:
31 g 35%ige Kaliumhydrid-Mineralölsuspension,
104 g 1,4,5-Tris-(4-chlorphenyl)-4-imidazolin-2-on,
600 ml Dimethylformamid,
65 g 8-Bromcaprylsäuremethylester.
Reinigung durch Säulenchromatographie (Kieselgel//Hexan/Essigsäureethylester)
Ausbeute: 12,8 g Fp. 94–96°C IR (in KBr): 1739 $cm^{-1}$

Beispiel 14

Darstellung von 11-(1,4,5-Triphenylimidazol-2-yloxy)-undecansäuremethylester analog Beispiel 1 aus:
36,3 g 35%ige Kaliumhydrid-Mineralölsuspension,
99 g 1,4,5-Triphenyl-4-imidazolin-2-on,
800 ml Dimethylformamid,
88 g 11-Bromundecansäuremethylester
Reinigung durch Säulenchromatographie (Kieselgel//Hexan/Essigsäureethylester)
Ausbeute: 35 g mit Fp. 79–80°C IR (in KBr): 1740 $cm^{-1}$

Analog den Beispielen 1–14 werden hergestellt:
1,4,5-Triphenylimidazol-2-yloxyessigsäureethylester,
3-(1,4,5-Triphenylimidazol-2-yloxy)-propionsäureethylester,
8-[1-(3,4-Dimethoxyphenyl)-4,5-diphenyl-imidazol-2-yloxy]-caprylsäuremethylester,
9-(1,4,5-Triphenylimidazol-2-yloxy)-pelargonsäuremethylester,
10-(1,4,5-Triphenylimidazol-2-yloxy)-caprinsäureethylester,
8-(1,4,5-Triphenylimidazol-2-yloxy)-caprylsäureethylester.

Beispiel 15

Darstellung von 8-(1,4,5-Triphenylimidazol-2-yloxy)-caprylsäure.

171 g 8-(1,4,5-Triphenylimidazol-2-yloxy)-caprylsäuremethylester und 44 g Natriumhydroxid werden in 1000 ml Methanol gelöst und die Mischung bei Raumtemperatur ca. 24 Stunden gerührt. Danach wird das Lösungsmittel im Vakuum abgezogen und der Rückstand mit Wasser aufgenommen. Die wässrige Lösung wird mit Ether gewaschen, mit verdünnter Salzsäure angesäuert, die ausgefallene Säure abgetrennt und getrocknet.
Ausbeute: 151 g mit Fp. 165°C MS [m/e] : 454 (19%), 312 (100%), 180 (17%)

Beispiel 16

Darstellung von 4-(1,4,5-Triphenylimidazol-2-yloxy)-buttersäure analog Beispiel 15 aus:
19,3 g 4-(1,4,5-Triphenylimidazol-2-yloxy)-buttersäuremethylester,
3,6 g Natriumhydroxid in
500 ml Dioxan und 50 ml Wasser
Ausbeute: 19 g mit Fp. 142°C MS [m/e] : 398 (0,8%), 312 (38%)

Beispiel 17

Darstellung von 5-(1,4,5-Triphenylimidazol-2-yloxy)-valeriansäure analog Beispiel 15 aus:
28 g 5-(1,4,5-Triphenylimidazol-2-yloxy)-valeriansäureethylester,
7,5 g Natriumhydroxid in 400 ml Dioxan und 70 ml Wasser
Ausbeute: 26,1 g mit Fp. 140°C MS [m/e] : 412 (2,6%), 312 (33%)

Beispiel 18

Darstellung von 6-(1,4,5-Triphenylimidazol-2-yloxy)-capronsäure analog Beispiel 15 aus:
28,5 g 6-(1,4,5-Triphenylimidazol-2-yloxy)-capronsäureethylester,
7,5 g Natriumhydroxid in
400 ml Dioxan und 70 ml Wasser
Ausbeute: 26,1 g mit Fp. 140–145°C MS [m/e] : 426 (0,9%), 312 (100%), 180 (29%)

Beispiel 19

Darstellung von 7-(1,4,5-Triphenylimidazol-2-yloxy)-önanthsäure analog Beispiel 15 aus:
2 g 7-(1,4,5-Triphenylimidazol-2-yloxy)-önanthsäureethylester,
0,6 g Natriumhydroxid in
150 ml Ethanol
Ausbeute: 1,5 g mit Fp. 149°C MS [m/e] : 440 (5%), 312 (100%), 180 (32%)

Beispiel 20

Darstellung von 8-[4,5-Diphenyl-1-(4-methoxyphenyl)-imidazol-2-yloxy]-caprylsäure analog Beispiel 15 aus:
25 g 8-[4,5-Diphenyl-1-(4-methoxyphenyl)-imidazol-2-yloxy]-caprylsäuremethylester,
8 g Natriumhydroxid in
250 ml Methanol.
Ausbeute: 20 g mit Fp. 132–133°C MS [m/e] : 484 (2,9%), 342 (100%), 210 (18%)

Beispiel 21

Darstellung von 8-[1-(4-Chlorphenyl)-4,5-diphenylimidazol-2-yloxy]-caprylsäure analog Beispiel 15 aus:
17,1 g 8-[1-(4-Chlorphenyl)-4,5-diphenylimidazol-2-yloxy]-caprylsäuremethylester
4 g Natriumhydroxid in
350 ml Dioxan und 50 ml Wasser.
Ausbeute: 14,5 g mit Fp. 118–120°C MS [m/e] : 488 (3,6%), 346 (100%), 310 (7%), 214 (18%)

Beispiel 22

Darstellung von 8-[4,5-Diphenyl-1-(4-methylphenyl)-imidazol-2-yloxy]-caprylsäure analog Beispiel 15 aus:
5,5 g 8-[4,5-Diphenyl-1-(4-methylphenyl)-imidazol-2-yloxy]-caprylsäuremethylester,
0,9 g Natriumhydroxid in
100 ml Dioxan und 10 ml Wasser.
Ausbeute: 2,8 g mit Fp. 124°C MS [m/e] : 468 (25%), 326 (100%), 312 (19%)

Beispiel 23

Darstellung von 8-[4,5-Diphenyl-1-(2-fluorphenyl)-imidazol-2-yloxy]-caprylsäure analog Beispiel 15 aus:
10 g 8-[4,5-Diphenyl-1-(2-fluorphenyl)-imidazol-2-yloxy]-caprylsäuremethylester,
1,7 g Natriumhydroxid in
200 ml Dioxan und 40 ml Wasser
Ausbeute: 8,2 g mit Fp. 81 °C

Beispiel 24
Darstellung von 8-[4,5-Bis-(4-chlorphenyl)-1-phenylimidazol-2-yloxy]-caprylsäure analog Beispiel 15 aus:
14,6 g 8-[4,5-Bis-(4-chlorphenyl)-1-phenyl-imidazol-2-yloxy]-caprylsäuremethylester,
2,5 g Natriumhydroxid in
300 ml Dioxan und 40 ml Wasser.
Ausbeute: 12,5 g mit Fp. 143–145°C MS [m/e] : 522 (0,7%), 380 (100%), 214 (24%)

Beispiel 25
Darstellung von 8-[4,5-Bis-(4-fluorphenyl)-1-phenyl-imidazol-2-yloxy]-caprylsäure analog Beispiel 15 aus:
18,5 g 8-[4,5-Bis-(4-fluorphenyl)-1-phenyl-imidazol-2-yloxy]-caprylsäuremethylester,
3 g Natriumhydroxid in
400 ml Dioxan und 40 ml Wasser.
Ausbeute: 14,5 g mit Fp. 155°C MS [m/e] : 490 (13%), 348 (100%), 305 (4,2%), 198 (18%)

Beispiel 26
Darstellung von 8-[4,5-Bis-(4-methoxyphenyl)-1-phenyl-imidazol-2-yloxy]-caprylsäure analog Beispiel 15 aus:
8,44 g 8-[4,5-Bis-(4-methoxyphenyl)-1-phenyl-imidazol-2-yloxy)-caprylsäuremethylester,
1,27 g Natriumhydroxid in
100 ml Dioxan und 10 ml Wasser.
Ausbeute: 5 g mit Fp. 147–149°C MS [m/e] : 514 (0,8%), 372 (100%), 357 (38%)

Beispiel 27
Darstellung von 8-[1,4,5-Tris-(4-chlorphenyl)-imidazol-2-yloxy]-caprylsäure analog Beispiel 15 aus:
12,8 g 8-[1,4,5-Tris-(4-chlorphenyl)-imidazol-2-yloxy]-caprylsäuremethylester,
3,6 g Natriumhydroxid in
450 ml Dioxan und 40 ml Wasser.
Ausbeute: 9,6 g mit Fp. 133–135°C
MS [m/e] : 416 /$M^+ - 142$, (100%)/, 378 (7%) 248 (13%), 138 (11%), 111 (19%)

Beispiel 28
Darstellung von 11-(1,4,5-Triphenylimidazol-2-yloxy)-undecansäure analog Beispiel 15 aus:
33 g 11-(1,4,5-Triphenylimidazol-2-yloxy)-undecansäuremethylester,
6 g Natriumhydroxid in
800 ml Ethanol.
Ausbeute: 28 g mit Fp. 107°C MS [m/e] : 496 (1%), 312 (100%), 180 (32%)

Analog den Beispielen 15–28 werden hergestellt:
1,4,5-Triphenylimidazol-2-yloxyessigsäure,
3-(1,4,5-Triphenylimidazol-2-yloxy)-propionsäure,
8-[1-(3,4-Dimethoxyphenyl)-4,5-diphenyl-imidazol-2-yloxy]-caprylsäure,
9-(1,4,5-Triphenylimidazol-2-yloxy)-pelargonsäure,
10-(1,4,5-Triphenylimidazol-2-yloxy)-caprinsäure.

Beispiel 29
Darstellung von 8-(1,4,5-Triphenylimidazol-2-yloxy)-caprylsäure-Natriumsalz.
8-(1,4,5-Triphenylimidazol-2-yloxy)-caprylsäure wird in Ethanol gelöst, die Lösung mit der äquivalenten Menge ethanolischer Natronlauge versetzt, die Mischung im Vakuum zur Trockne eingeengt und der Rückstand pulverisiert.
Ausbeute: quantitativ IR (in KBr): 1553 $cm^{-1}$
Analog dem Beispiel 29 werden folgende Natriumsalze hergestellt:

Beispiel 30
1,4,5-Triphenylimidazol-2-yloxyessigsäure-Natriumsalz IR (in KBr): 1615 $cm^{-1}$

Beispiel 31
3-(1,4,5-Triphenylimidazol-2-yloxy)-propionsäure-Natriumsalz
IR (in KBr): 1560 $cm^{-1}$

Beispiel 32
4-(1,4,5-Triphenylimidazol-2-yloxy)-buttersäure-Natriumsalz
IR (in KBr): 1552 $cm^{-1}$

Beispiel 33
5-(1,4,5-Triphenylimidazol-2-yloxy)-valeriansäure-Natriumsalz
IR (in KBr): 1554 $cm^{-1}$

Beispiel 34
6-(1,4,5-Triphenylimidazol-2-yloxy)-capronsäure-Natriumsalz
IR (in KBr): 1553 $cm^{-1}$

Beispiel 35
7-(1,4,5-Triphenylimidazol-2-yloxy)-önanthsäure-Natriumsalz
IR (in KBr): 1551 $cm^{-1}$

Beispiel 36
8-[4,5-Diphenyl-1-(4-methoxyphenyl)-imidazol-2-yloxy]-caprylsäure-Natriumsalz
IR (in KBr): 1551 $cm^{-1}$

Beispiel 37
8-[1-(4-Chlorphenyl)-4,5-diphenylimidazol-2-yloxy]-caprylsäure-Natriumsalz
IR (in KBr): 1555 $cm^{-1}$

Beispiel 38
8-[4,5-Diphenyl-1-(4-methylphenyl)-imidazol-2-yloxy]-caprylsäure-Natriumsalz
IR (in KBr): 1553 $cm^{-1}$

Beispiel 39

8-[4,5-Diphenyl-1-(2-fluorphenyl)-imidazol-2-yloxy]-caprylsäure-Natriumsalz

IR (in KBr): 1555 $cm^{-1}$

Beispiel 40

8-[4,5-Bis-(4-chlorphenyl)-1-phenyl-imidazol-2-yloxy]-caprylsäure-Natriumsalz

IR (in KBr): 1560 $cm^{-1}$

Beispiel 41

8-[4,5-Bis-(4-fluorphenyl)-1-phenyl-imidazol-2-yloxy]-caprylsäure-Natriumsalz

IR (in KBr): 1556 $cm^{-1}$

Beispiel 42

8-[4,5-Bis-(4-methoxyphenyl)-1-phenyl-imidazol-2-yloxy]-caprylsäure-Natriumsalz

IR (in KBr): 1553 $cm^{-1}$

Beispiel 43

8-[1,4,5-Tris-(4-chlorphenyl)-imidazol-2-yloxy]-caprylsäure-Natriumsalz

IR (in KBr): 1563 $cm^{-1}$

Beispiel 44

8-[1-(3,4-Dimethoxyphenyl)-4,5-diphenyl-imidazol-2-yloxy]-caprylsäure-Natriumsalz

IR (in KBr): 1555 $cm^{-1}$

Beispiel 45

9-(1,4,5-Triphenylimidazol-2-yloxy)-pelargonsäure-Natriumsalz

IR (in KBr): 1558 $cm^{-1}$

Beispiel 46

10-(1,4,5-Triphenylimidazol-2-yloxy)-caprinsäure-Natriumsalz

IR (in KBr): 1560 $cm^{-1}$

Beispiel 47

11-(1,4,5-Triphenylimidazol-2-yloxy)-undecansäure-Natriumsalz

IR (in KBr): 1565 $cm^{-1}$

Beispiel 48

Darstellung des 8-(1,4,5-Triphenylimidazol-2-yloxy)-caprylsäurehexylesters.

4,8 g 8-(1,4,5-Triphenylimidazol-2-yloxy)-caprylsäure-Natriumsalz, 1,2 g 1-Chlorhexan, 0,2 g Natriumjodid in 20 ml Dimethylformamid werden 8 Stunden auf 80°C erhitzt. Nach dem Abkühlen wird mit Wasser verdünnt und mit Chloroform extrahiert. Die Chloroformlösung wird nacheinander mit Wasser, 5%iger Natriumhydrogencarbonatlösung und Wasser gewaschen, über Natriumsulfat getrocknet und im Vakuum eingeengt. Der Rückstand wird durch Säulenchromatographie (Kieselgel//Hexan/Essigsäureethylester) gereinigt.

Ausbeute: 4,1 g mit Fp. 92°C IR (im KBr): 1726 $cm^{-1}$

Beispiel 49

Darstellung des 8-(1,4,5-Triphenylimidazol-2-yloxy)-caprylsäurebenzylesters analog Beispiel 48 aus:

4,8 g 8-(1,4,5-Triphenylimidazol-2-yloxy)-caprylsäure-Natriumsalz

1,3 g Benzylchlorid, 0,2 g Natriumjodid in 20 ml Dimethylformamid.

Ausbeute: 4,6 g mit Fp. 95°C IR (im KBr): 1731 $cm^{-1}$

**Patentansprüche für die Vertragsstaaten: BE, CH, DE, FR, GB, IT; LI, LU, NL, SE**

1. ω-(1,4,5-Triphenylimidazol-2-yloxy)-alkansäuren und ihre Derivate der allgemeinen Formel I

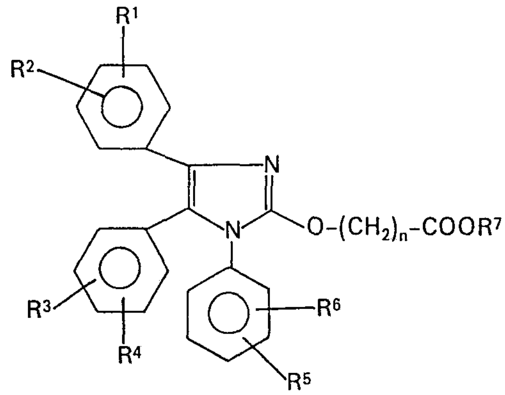

I

worin n eine ganze Zahl von 1 bis 10 ist, $R^1$, $R^2$, $R^3$, $R^4$, $R^5$, $R^6$: gleich oder verschieden sein können und unabhängig voneinander Wasserstoff, Halogen, $C_{1-3}$-Alkyl, $C_{1-2}$-Alkoxy, Trifluormethyl oder 2 Reste zusammen Methylendioxy bedeuten, $R^7$: Wasserstoff, ein Alkaliion oder eine geradkettige bzw. verzweigte Alkylgruppe mit 1–6 Kohlenstoffatomen oder einen Benzylrest bedeutet.

2. 1,4,5-Triphenylimidazol-2-yloxyessigsäure und deren pharmazeutisch verträgliche Salze und Ester.

3. 4-(1,4,5-Triphenylimidazol-2-yloxy)-buttersäure und deren pharmazeutisch verträgliche Salze und Ester.

4. 5-(1,4,5-Triphenylimidazol-2-yloxy)-valeriansäure und deren pharmazeutisch verträgliche Salze und Ester.

5. 6-(1,4,5-Triphenylimidazol-2-yloxy)-capronsäure und deren pharmazeutisch verträgliche Salze und Ester.

6. 7-(1,4,5-Triphenylimidazol-2-yloxy)-önanthsäure und deren pharmazeutisch verträgliche Salze und Ester.

7. 8-(1,4,5-Triphenylimidazol-2-yloxy)-caprylsäure und deren pharmazeutisch verträgliche Salze und Ester.

8. 8-[4,5-Diphenyl-1-(4-methoxyphenyl)-imidazol-2-yloxy]-caprylsäure und deren pharmazeutisch verträgliche Salze und Ester.

9. 8-[1-(4-Chlorphenyl)-4,5-diphenylimidazol-2-yloxy]-caprylsäure und deren pharmazeutisch verträgliche Salze und Ester.

10. 8-[4,5-Diphenyl-1-(4-methylphenyl)-imidazol-2-yloxy]-caprylsäure und deren pharmazeutisch verträgliche Salze und Ester.

11. 8-[4,5-Diphenyl-1-(2-fluorphenyl)-imidazol-2-yloxy]-caprylsäure und deren pharmazeutisch verträgliche Salze und Ester.

12. 8-[4,5-Bis-(4-chlorphenyl)-1-phenyl-imidazol-2-yloxy]-caprylsäure und deren pharmazeutisch verträgliche Salze und Ester.

13. 8-[4,5-Bis-(4-fluorphenyl)-1-phenyl-imidazol-2-yloxy]-caprylsäure und deren pharmazeutisch verträgliche Salze und Ester.

14. 8-[4,5-Bis-(4-methoxyphenyl)-1-phenyl-imidazol-2-yloxy]-caprylsäure und deren pharmazeutisch verträgliche Salze und Ester.

15. 8-[1,4,5-Tris-(4-chlorphenyl)-imidazol-2-yloxy]-caprylsäure und deren pharmazeutisch verträgliche Salze und Ester.

16. 8-[1-(3,4-Dimethoxyphenyl)-4,5-diphenyl-imidazol-2-yloxy]-caprylsäure und deren pharmazeutisch verträgliche Salze und Ester.

17. 9-(1,4,5-Triphenylimidazol-2-yloxy)-pelargonsäure und deren pharmazeutisch verträgliche Salze und Ester.

18. 10-(1,4,5-Triphenylimidazol-2-yloxy)-caprinsäure und deren pharmazeutisch verträgliche Salze und Ester.

19. 11-(1,4,5-Triphenylimidazol-2-yloxy)-undecansäure und deren pharmazeutisch verträgliche Salze und Ester.

20. Pharmazeutische Präparate, dadurch gekennzeichnet, dass sie eine Verbindung der Formel I gemäss den Ansprüchen 1–19 als Wirkstoff im Gemisch mit üblichen pharmazeutischen Hilfs- und Trägerstoffen enthalten.

**Patentanspruch**

Verfahren zur Herstellung der Verbindungen der Ansprüche 1–19, dadurch gekennzeichnet, dass man ein 4-Imidazolin-2-on der allgemeinen Formel II

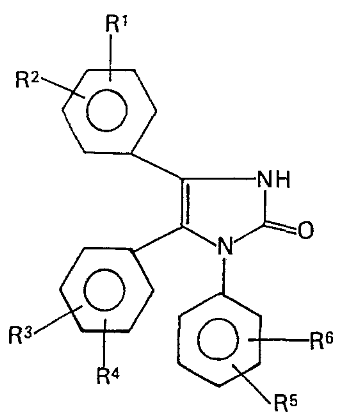

II

wobei $R^1$, $R^2$, $R^3$, $R^4$, $R^5$, $R^6$ die in Formel I angegebene Bedeutung haben, in einem geeigneten organischen Lösungsmittel unter Zusatz einer Hilfsbase bzw. nach Überführung in ein geeignetes Salz mit einem Alkylierungsmittel der allgemeinen Formel III umsetzt,

$X-(CH_2)_n-COOR^7$ III

wobei n und $R^7$ die in Formel I angegebene Bedeutung besitzen und X ein Halogen- oder Tosylrest ist, die erhaltenen Ester von den in Folge N-Alkylierung von II entstehenden Isomeren trennt und die so erhaltenen Ester der allgemeinen Formel I mit $R^7 = C_{1-6}$-Alkyl oder Benzyl in die Säuren der Formel I mit $R^7 = H$ und diese in die Alkalisalze der Formel I mit $R^7$ = Alkali überführt oder die erhaltenen Säuren der allgemeinen Formel I mit $R^7 = H$ oder die Alkalisalze der allgemeinen Formel I mit $R^7$ = Alkali in die Ester der Formel I mit $R^7 = C_{1-6}$-Alkyl oder Benzyl überführt.

**Patentanspruch für den Vertragsstaat: Österreich**

Verfahren zur Herstellung von ω-(1,4,5-Triphenylimidazol-2-yloxy)-alkansäure und ihre Derivate der allgemeinen Formel I

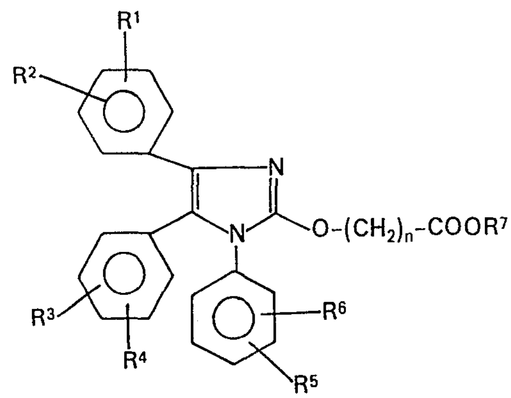

I

worin n eine ganze Zahl von 1 bis 10 ist, $R^1$, $R^2$, $R^3$, $R^4$, $R^5$, $R^6$ gleich oder verschieden sein können und unabhängig voneinander Wasserstoff, Halogen, $C_{1-3}$-Alkyl, $C_{1-2}$-Alkyloxy, Trifluormethyl oder zwei Reste zusammen Methylendioxy bedeuten, $R^7$ Wasserstoff, ein Alkaliion oder eine geradkettige bzw. verzweigte Alkylgruppe mit 1 bis 6 Kohlenstoffatomen oder einen Benzylrest bedeutet, dadurch gekennzeichnet, dass man ein 4-Imidazolin-2-on der allgemeinen Formel II

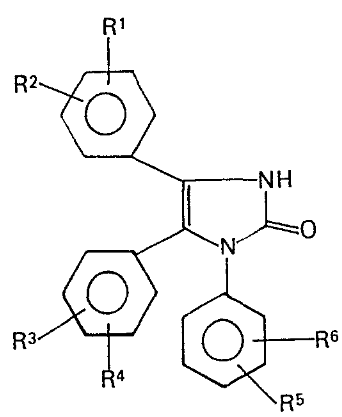

II

wobei $R^1$, $R^2$, $R^3$, $R^4$, $R^5$, $R^6$ die in Formel I angegebene Bedeutung haben, in einem geeigneten organischen Lösungsmittel unter Zusatz einer Hilfsbase bzw. nach Überführung in ein geeignetes

Salz mit einem Alkylierungsmittel der allgemeinen Formel III umsetzt.

$X-(CH_2)_n-COOR^7$ III

wobei n und $R^7$ die in Formel I angegebene Bedeutung besitzen und X ein Halogen- oder Tosylrest ist, die erhaltenen Ester von den in Folge N-Alkylierung von II entstehenden Isomeren trennt und die so erhaltenen Ester der allgemeinen Formel I mit $R^7 = C_{1-6}$-Alkyl oder Benzyl in die Säuren der Formel I mit $R^7$ = H und diese in die Alkalisalze der Formel I mit $R^7$ = Alkali überführt oder die erhaltenen Säuren der allgemeinen Formel I mit $R^7$ = H oder die Alkalisalze der allgemeinen Formel I mit $R^7$ = Alkali in die Ester der Formel I mit $R^7 = C_{1-6}$-Alkyl oder Benzyl überführt.

**Claims for the Contracting states: BE, CH/LI, DE, FR, GB, IT, LU, NL, SE**

1. ω-(1.4.5-Triphenylimidazol-2-yloxy)-alkanoic acids and their derivates, of the general formula I

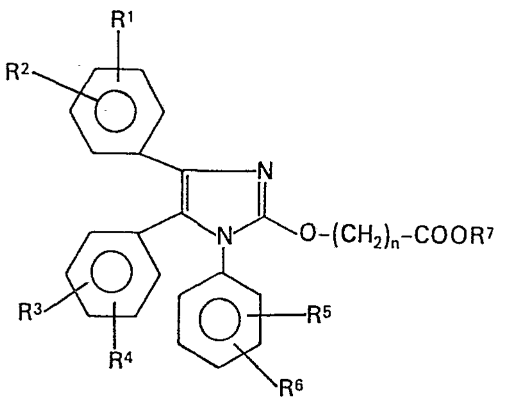

I

wherein n is an integer from 1 to 10, $R^1$, $R^2$, $R^3$, $R^4$, $R^5$, $R^6$ can be identical or different and independently of one another denote hydrogen, halogen, $C_{1-3}$-alkyl, $C_{1-2}$-alkoxy, trifluoromethyl, or 2 groups together are methylenedioxy and $R^7$ denotes hydrogen, an alkali metal ion, a straight-chain or branched alkyl group with 1-6 carbon atoms or a benzyl group.

2. 1.4.5-Triphenylimidazol-2-yloxyacetic acid and pharmaceutically acceptable salts and esters thereof.

3. 4-(1.4.5-Triphenylimidazol-2-yloxy)-butyric acid and pharmaceutically acceptable salts and esters thereof.

4. 5-(1.4.5-Triphenylimidazol-2-yloxy)-valeric acid and pharmaceutically acceptable salts and esters thereof.

5. 6-(1.4.5-Triphenylimidazol-2-yloxy)-caproic acid and pharmaceutically acceptable salts and esters thereof.

6. 7-(1.4.5-Triphenylimidazol-2-yloxy)-oenanthic acid and pharmaceutically acceptable salts and esters thereof.

7. 8-(1.4.5-Triphenylimidazol-2-yloxy)-caprylic acid and pharmaceutically acceptable salts and esters thereof.

8. 8-[4.5-Diphenyl-1-(4-methoxyphenyl)-imidazol-2-yloxy]-caprylic acid and pharmaceutically acceptable salts and esters thereof.

9. 8-[1-(4-Chlorophenyl)-4.5-diphenylimidazol-2-yloxy]-caprylic acid and pharmaceutically acceptable salts and esters thereof.

10. 8-[4.5-Diphenyl-1-(4-methylphenyl)-imidazol-2-yloxy]-caprylic acid and pharmaceutically acceptable salts and esters thereof.

11. 8-[4.5-Diphenyl-1-(2-fluorophenyl)-imidazol-2-yloxy]-caprylic acid and pharmaceutically acceptable salts and esters thereof.

12. 8-[4.5-Bis-(4-chlorophenyl)-1-phenyl-imidazol-2-yloxy]-caprylic acid and pharmaceutically acceptable salts and esters thereof.

13. 8-[4.5-Bis-(4-fluorophenyl)-1-phenyl-imidazol-2-yloxy]-caprylic acid and pharmaceutically acceptable salts and esters thereof.

14. 8-[4.5-Bis-(4-methoxyphenyl)-1-phenyl-imidazol-2-yloxy]-caprylic acid and pharmaceutically acceptable salts and esters thereof.

15. 8-[1.4.5-Tris-(4-chlorophenyl)-imidazol-2-yloxy]-caprylic acid and pharmaceutically acceptable salts and esters thereof.

16. 8-[1-(3.4-Dimethoxyphenyl)-4,5-diphenylimidazol-2-yloxy]-caprylic acid and pharmaceutically acceptable salts and esters thereof.

17. 9-(1.4.5-Triphenylimidazol-2-yloxy)-pelargonic acid and pharmaceutically acceptable salts and esters thereof.

18. 10-(1.4.5-Triphenylimidazol-2-yloxy)-capric acid and pharmaceutically acceptable salts and esters thereof.

19. 11-(1.4.5-Triphenylimidazol-2-yloxy)-undecanoic acid and pharmaceutically acceptable salts and esters thereof.

20. Process for producing compounds of claims 1 to 19 characterised in that a 4-imidazolin-2-one of the general formula II

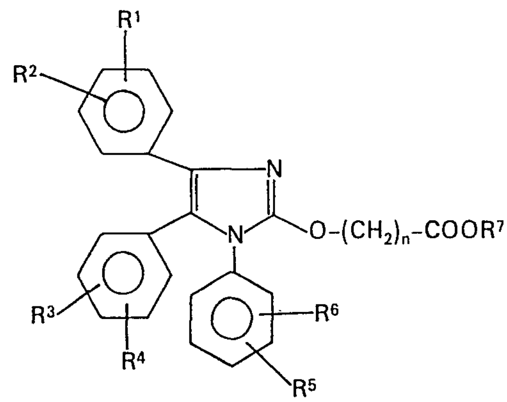

II

wherein $R^1$, $R^2$, $R^3$, $R^4$, $R^5$ and $R^6$ have the meaning given in formula I, is subjected to reaction with an alkylating agent of the general formula III

$X-(CH_2)_n-COOR^7$ III

wherein n and $R^7$ have the meaning given in formula I and X is a halogen atom or a tosyl residue,

in a suitable organic solvent with the addition of an auxiliary base or after conversion of the 4-imidazolin-2-one of formula II into a suitable acid addition salt, the resulting ester is separated from the resulting isomeres resulting from N-alkylation and the thus obtained esters of general formula I with $R^7 = C_{1-6}$-alkyl or benzyl are converted into the acids of formula I with $R^7$ = H and these acids are converted into the alkali metal salts of formula I with $R^7$ = alkali metal or the resulting acids of general formula I with $R^7$ = H or the alkali metal salts of general formula I with $R^7$ = alkali metal are converted into the esters of formula I with $R^7 = C_{1-6}$-alkyl or benzyl.

21. Pharmaceutical preparations, characterised in that they contain a compound of the formula I according to claims 1–19 as the active compound, mixed with usual pharmaceutical auxiliary and carrier agents.

**Claim for the contracting state: AT**

1. Process for producing ω-(1.4.5-Triphenylimidazol-2-yloxy)-alkanoic acids and their derivatives of the general formula I

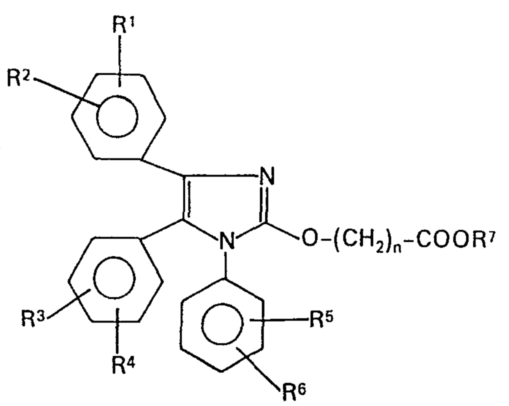

I

wherein n is an integer from 1 to 10, $R^1$, $R^2$, $R^3$, $R^4$, $R^5$, $R^6$ can be identical or different and independently of one another denote hydrogen, halogen, $C_{1-3}$-alkyl, $C_{1-2}$-alkoxy, trifluoromethyl, or 2 groups together are methylenedioxy and $R^7$ denotes hydrogen, an alkali metal ion, a straight-chain or branched alkyl group with 1–6 carbon atoms or a benzyl group, characterised in that a 4-imidazolin-2-one of the general formula II

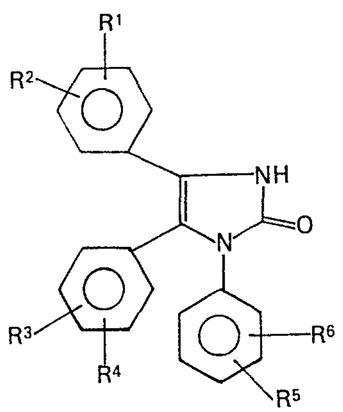

II

wherein $R^1$, $R^2$, $R^3$, $R^4$, $R^5$ and $R^6$ have the meaning given in formula I, is subjected to reaction with an alkylating agent of the general formula III

$$X\text{-}(CH_2)_n\text{-}COOR^7 \qquad III$$

wherein n and $R^7$ have the meaning given in formula I and X is a halogen atom or a tosyl residue, in a suitable organic solvent with the addition of an auxiliary base or after conversion of the 4-imidazolin-2-one of formula II into a suitable acid addition salt, the resulting ester is separated from the resulting isomeres resulting from N-alkylation and the thus obtained esters of general formula I with $R^7 = C_{1-6}$-alkyl or benzyl are converted into the acids of formula I with $R^7$ = H and these acids are converted into the alkali metal salts of formula I with $R^7$ = alkali metal or the resulting acids of general formula I with $R^7$ = H or the alkali metal salts of general formula I with $R^7$ = alkali metal are converted into the esters of formula I with $R^7 = C_{1-6}$-alkyl or benzyl.

**Revendications pour les Etats contractants: BE, CH/LI, DE, FR, GB, IT, LU, NL, SE**

1. Acides ω-(1,4,5-triphénylimidazol-2-yloxy)-alcanoïques et leurs dérivés de formule générale I

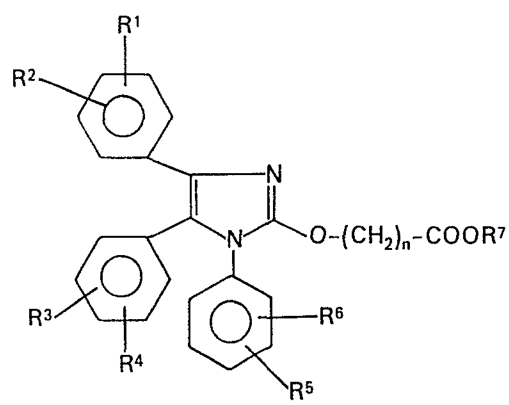

I

où n est un nombre entier de 1 à 10, $R^1$, $R^2$, $R^3$, $R^4$, $R^5$, $R^6$: peuvent être identiques ou différents et indépendamment les uns des autres représentent un hydrogène, un halogène, un alkyle $C_{1-3}$, un alcoxy $C_{1-2}$, un trifluorométhyle ou 2 résidus formant ensemble le méthylènedioxy, $R^7$: représente un hydrogène, un ion alcalin ou un groupe alkyle à chaîne droite ou ramifiée avec 1–6 atomes de carbone ou un résidu benzyle.

2. Acide 1,4,5-triphénylimidazol-2-yloxyacétique et ses sels et esters pharmaceutiquement tolérés.

3. Acide 4-(1,4,5-triphénylimidazol-2-yloxy)-butyrique et ses sels et esters pharmaceutiquement tolérés.

4. Acide 5-(1,4,5-triphénylimidazol-2-yloxy)-valérique et ses sels et esters pharmaceutiquement tolérés.

5. Acide 6-(1,4,5-triphénylimidazol-2-yloxy)-caproïque et ses sels et esters pharmaceutiquement tolérés.

6. Acide 7-(1,4,5-triphénylimidazol-2-yloxy)-œnanthoïque et ses sels et esters pharmaceutiquement tolérés.

7. Acide 8-(1,4,5-triphénylimidazol-2-yloxy)-caprylique et ses sels et esters pharmaceutiquement tolérés.

8. Acide 8-[4,5-diphényl-1-(méthoxyphényl)-imidazol-2-yloxy]-caprylique et ses sels et esters pharmaceutiquement tolérés.

9. Acide 8-[1-(4-chlorophényl)-4,5-diphényl-imidazol-2-yloxy]-caprylique et ses sels et esters pharmaceutiquement tolérés.

10. Acide 8-[4,5-diphényl-1-(4-méthylphényl)-imidazol-2-yloxy]-caprylique et ses sels et esters pharmaceutiquement tolérés.

11. Acide 8-[4,5-diphényl-1-(2-fluorophényl)-imidazol-2-yloxy]-caprylique et ses sels et esters pharmaceutiquement tolérés.

12. Acide 8-[4,5-bis-(4-chlorophényl)-1-phényl-imidazol-2-yloxy]-caprylique et ses sels et esters pharmaceutiquement tolérés.

13. Acide 8-[4,5-bis-(4-fluorophényl-1-phényl-imidazol-2-yloxy]-capryliques et ses sels et esters pharmaceutiquement tolérés.

14. Acide 8-[4,5-bis-(4-méthoxyphényl)-1-phényl-imidazol-2-yloxy]-caprylique et ses sels et esters pharmaceutiquement tolérés.

15. Acide 8-[1,4,5-tris-(4-chlorophényl)-imidazol-2-yloxy]-caprylique et ses sels et esters pharmaceutiquement tolérés.

16. Acide 8-[1-(3,4-diméthoxyphényl)-4,5-diphényl-imidazol-2-yloxy]-caprylique et ses sels et esters pharmaceutiquement tolérés.

17. Acide 9-(1,4,5-triphénylimidazol-2-yloxy)-pélargonique et ses sels et esters pharmaceutiquement tolérés.

18. Acide 10-(1,4,5-triphénylimidazol-2-yloxy)-caprique et ses sels et esters pharmaceutiquement tolérés.

19. Acide 11-(1,4,5-triphénylimidazol-2-yloxy)-undécanoïque et ses sels et esters pharmaceutiquement tolérés.

20. Procédé de préparation des composés des revendications 1 à 19, caractérisés en ce qu'on fait réagir une 4-imidazolin-2-one de formule générale II

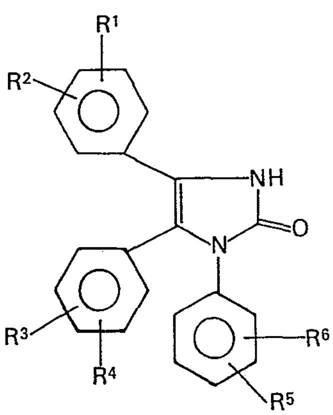

II

où $R^1$, $R^2$, $R^3$, $R^4$, $R^5$, $R^6$ ont la signification donnée dans la formule I, dans un solvant organique approprié avec addition d'une base auxiliaire ou après transformation en un sel approprié, avec un agent alkylant de formule générale III

$$X\text{-}(CH_2)_n\text{-}COOR^7 \qquad III$$

où n et $R^7$ possèdent la signification donnée dans la formule I et X est un résidu d'halogène ou de tosyle, on sépare les esters obtenus d'une façon connue en soi des isomères formés par suite de la N-alkylation de II et on transforme les esters ainsi obtenus de formule générale I avec $R^7$ = alkyle $C_{1-6}$ ou benzyle d'une façon connue en soi en acides de formule I avec $R^7$ = H et on transforme ceux-ci en sels alcalins de formule I avec $R^7$ = alcali ou bien on transforme les acides obtenus de formule générale I avec $R^7$ = H ou les sels alcalins de formule générale I avec $R^7$ = alcali d'une façon connue en soi en esters de formule I avec $R^7$ = alkyle $C_{1-6}$ ou benzyle.

21. Préparations pharmaceutiques caractérisées en ce qu'elle contiennent un composé de formule I conformément aux revendications 1 à 19 en tant que principe actif en mélange avec des adjuvants et des excipients pharmaceutiques habituels.

**Revendication pour l'Etat contractant: AT**

Procédé de préparation d'acides ω-(1,4,5-triphénylimidazol-2-yloxy)-alcanoïques et de leurs dérivés de formule générale I

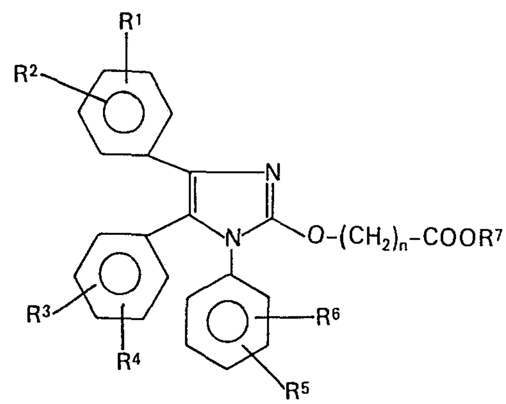

I

où n est un nombre entier de 1 à 10, $R^1$, $R^2$, $R^3$, $R^4$, $R^5$, $R^6$ peuvent être identiques ou différents et indépendamment les uns des autres représentent un hydrogène, un halogène, un alkyle $C_{1-3}$, un alkyloxy $C_{1-2}$, un trifluorométhyle ou deux résidus formant ensemble le méthylènedioxy, $R^7$ représente un hydrogène, un ion alcalin ou un groupe alkyle à chaîne droite ou ramifiée avec 1 à 6 atomes de carbone ou un résidu benzyle, caractérisé en ce qu'on fait réagir une 4-imidazolin-2-one de formule générale II

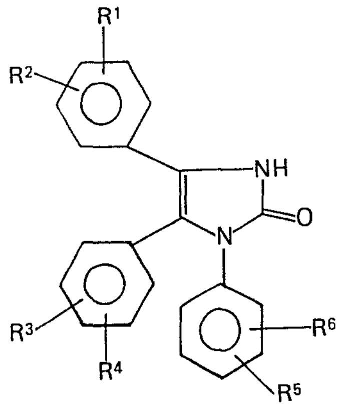

II

où $R^1$, $R^2$, $R^3$, $R^4$, $R^5$, $R^6$, ont la signification donnée dans la formule I, dans un solvant organique approprié avec addition d'une base auxiliaire ou après transformation en un sel approprié, avec un agent alkylant de formule générale III

$$X-(CH_2)_n-COOR^7 \quad \text{III}$$

où n et $R^7$ possèdent les significations données dans la formule I et X est un résidu d'halogène ou de tosyle, et on transforme les esters ainsi obtenus de formule générale I avec $R^7$ = alkyle $C_{1-6}$ ou benzyle d'une façon connue en soi en acides de formule I avec $R^7$ = H et ceux-ci en sels alcalins de formule I avec $R^7$ = alcali ou bien on transforme les acides obtenus de formule générale I avec $R^7$ = H ou les sels alcalins de formule I avec $R^7$ = alcali d'une façon connue en soi en esters de formule I avec $R^7$ = alkyle $C_{1-6}$ ou benzyle.